# EUROPÄISCHE PATENTSCHRIFT

(11) **EP 0 780 397 B1**
(45) Veröffentlichungstag und Bekanntmachung des Hinweises auf die Patenterteilung: **10.06.1998**
(21) Anmeldenummer: 96120293.4
(22) Anmeldetag: 18.12.1996
(51) Int. Cl.: C07J 41/00, A61K 31/565

(54) **Neue 17alpha-Cyanomethylestra-4,9-dien-derivate, Verfahren zu ihrer Herstellung und diese Verbindungen enthaltende pharmazeutische Zusammensetzungen**
New 17alpha-Cyanomethylestra-4,9-dien-derivatives, a process for the production of these compounds and pharmaceutical compositions containing them
Nouveaux dérivés 17alpha-cyanométhylestra-4,9-diène, un procédé pour leur préparation et compositions pharmaceutiques les renfermant

(30) Priorität: 22.12.1995 DE 19548449; 22.12.1995 DE 19548450
(43) Veröffentlichungstag der Anmeldung: 25.06.1997
(73) Patentinhaber: Jenapharm GmbH & Co. KG, 07745 Jena (DE)
(72) Erfinder: Teichmüller, Gerhard, 07743 Jena (DE); Müller, Gerd, 07745 Jena (DE); Schwarz, Sigfrid, Prof.-Dr., 07743 Jena (DE); Undeutsch, Bernd, Dr., 07745 Jena (DE); Henkel, Harry, 99510 Apolda (DE); Gebühr, Ronald, 07743 Jena (DE); Kaufmann, Günter, Dr., 07743 Jena (DE); Hübler, Doris, Dr., 07407 Schmieden (DE); Oettel, Michael, Prof.-Dr., 07743 Jena (DE)

(56) Entgegenhaltungen:
- DE-A- 1 966 921
- DE-A- 2 718 872
- US-A- 3 337 537
- US-A- 3 904 611
- US-A- 3 907 844
- US-A- 3 961 053
- CHEMICAL ABSTRACTS, vol. 123, no. 21, 20.November 1995 Columbus, Ohio, US; abstract no. 286389, WANG Z ET AL: "Preparation and of trifluoromethyl steroids as contraceptives" Seite 1246; Spalte 2; XP002031648 & CN 1 097 763 A (SHANGHAI ORGANIC CHEMISTRY INSTITUTE, CHINESE ACADEMY OF SCIENCES) 25.Januar 1995 & DATABASE WPI Section Ch, Week 9720 Derwent Publications Ltd., London, GB; Class B01, AN 97-213535 & CN 1 097 763 , 25.Januar 1995
- JOURNAL OF STEROID BIOCHEMISTRY, Bd. 22, Nr. 3, 1985, Seiten 289-292, XP002031647 C. H. SPILMAN ET AL: "Relationship Between Progesterone Receptor Binding Affinity and Progestin Biological Activity"

## Beschreibung

Die Erfindung betrifft neue 17α-Cyanomethylestra-4,9-dien-derivate der allgemeinen Formel I 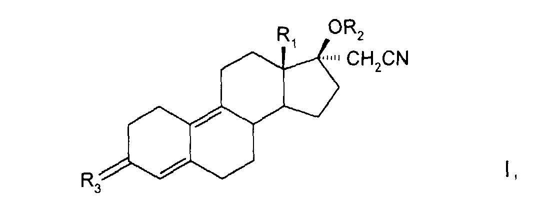 worin
R₁ eine Alkylgruppe mit 1 bis 2 Kohlenstoffatomen, R₂ ein Acylrest mit 1 bis 10 Kohlenstoffatomen,
R₃ ein Sauerstoffatom oder eine R-O-N-Gruppierung, wobei R ein Wasserstoffatom, ein Acylrest mit 1 bis 10 Kohlenstoffatomen, ein Alkylrest mit 1 bis 10 Kohlenstoffatomen, ein Sulfamoylrest oder Trialkylsilylrest darstellen, bedeuten.

Aus der Fach- und Patentliteratur (DE 27 18 872) ist das 17α-Cyanomethyl-17β-hydroxy-estra-4,9-dien-3-on (DIENOGEST) bekannt. Die erfindungsgemäßen Verbindungen sind neu, ihre Darstellung und ihre biologische Wirsamkeit wurden bisher noch nicht beschrieben.

Der Erfindung liegt die Aufgabe zugrunde, neue 17α-Cyanomethylestra-4,9-dien-derivate zur Verfügung zu stellen sowie Verfahren zu ihrer Herstellung aufzuzeigen.

Die Aufgabe wird erfindungsgemäß dadurch gelöst, daß neue 17α-Cyanomethylestra-4,9-dien-derivate, wie im Anspruch 1 dargestellt, hergestellt werden.

Erfindungsgemäße bevorzugte Verbindungen sind beispielsweise :
17α-Cyanomethylestra-4,9-dien-3-on-17β-yl-acetat,
17α-Cyanomethylestra-4,9-dien-3-on-17β-yl-propionat,
17α-Cyanomethylestra-4,9-dien-3-on-17β-yl-butyrat,
17α-Cyanomethylestra-4,9-dien-3-on -17β-yl-iso-butyrat,
17α-Cyanomethylestra-4,9-dien-3-on-17β-yl-valerat,
17α-Cyanomethylestra-4,9-dien-3-on-17β-yl-iso-valerat,
17α-Cyanomethylestra-4,9-dien-3-on-17β-yl-decanoat,
17α-Cyanomethylestra-4,9-dien-3-on-17β-yl-cyclohexanoat,
17α-Cyanomethylestra-4,9-dien-3-on-17β-yl-benzoat,
17α-Cyanomethyl-3-hydroxyiminoestra-4,9-dien-17β-yl-acetat,
17α-Cyanomethyl-3-hydroxyiminoestra-4,9-dien-17β-yl-propionat,
17α-Cyanomethyl-3-hydroxyiminoestra-4,9-dien-17β-yl-butyrat,
17α-Cyanomethyl-3-hydroxyiminoestra-4,9-dien-17β-yl-valerat,
17α-Cyanomethyl-3-hydroxyiminoestra-4,9-dien-17β-yl-cyclo-pentanoat,
17α-Cyanomethyl-3-hydroxyiminoestra-4,9-dien-17β-yl-benzoat,
17α-Cyanomethyl-3-hydroxyiminoestra-4,9-dien-17β-yl-decanoat,
3-Acetoxyimino-17α-cyanomethylestra-4,9-dien-17β-yl-acetat,
3-Acetoxyimino-17α-cyanomethylestra-4,9-dien-17β-yl-valerat,
3-Acetoxyimino-17α-cyanomethylestra-4,9-dien-17β-yl-benzoat,
17α-Cyanomethyl-3-propionyloxyimino-estra-4,9-dien-17β-yl-acetat,
3-Benzoyloxyimino -17α-cyanomethylestra-4,9-dien-17β-yl-acetat,
3-Benzoyloxyimino -17α-cyanomethylestra-4,9-dien-17β-yl-benzoat,
3-tert-Butoxyimino-17α-cyanomethyl-estra-4,9-dien-17β-yl-acetat,
3-tert-Butoxyimino -17α-cyanomethyl-estra-4,9-dien-17β-yl-benzoat,
17α-Cyanomethyl-3-( N,N-diethylsulfamoyloxy )-imino-estra-4,9-dien-17β-yl-acetat,
17α-Cyanomethyl-3-(N-acetylsulfamoyloxy )-imino-estra-4,9-dien-17β-yl-valerianat.

Gegenstand der vorliegenden Verbindung sind ferner pharmazeutische Zusammensetzungen, die als Wirkstoff mindestens ein 17α-Cyanomethylestra-4,9-dien-derivat der allgemeinen Formel I enthalten, wobei diese Zusammensetzungen gegebenenfalls geeignete Hilfs-und Trägerstoffe enthalten.

Die erfindungsgemäßen Verbindungen besitzen im Vergleich zu konventionellen Gestagenen ein gestagenes Wirkprofil ohne unerwünschte Nebenwirkungen.
Sie verfügen über eine relativ starke gestagene Wirksamkeit ohne androgener und anaboler Restwirkung, dafür aber überraschenderweise signifikannter antiandrogener Partialwirkung.

Besonders vorteilhaft ist die ausgezeichnete Verträglichkeit der erfindungsgemäßen Verbindungen, die auch im Vergleich zu den konventionellen 17α-Ethinyl-17β-hydroxysteroiden auch bei erhöhter Dosierung kaum unerwünschte Nebenwirkungen hervorrufen.

Die pharmakologischen Untersuchungen zeigen, daß sich diese neuen 17α-Cyanomethyl-19-nor-Gestagene im Wirkprofil wesentlich von den herkömmlichen 17α-Ethinyl-19-nor-Gestagenen unterscheiden.

Mittels Clauberg-McPhail-Assay wurden die erfindungsgemäßen Verbindungen im Vergleich zu Dienogest am Kaninchen geprüft.
Der Clauberg-McPhail-Assay ist nach EDGREN, R.A., 'Pharmacology of the Contraceptive Steroids', Goldzieher and Fotherby, Raven Press 1994, ein anerkannter Test zur Bestimmung der transformatorischen Aktivität von Gestagenen am Kaninchenendometrium, der mit der transformatorischen Aktivität von Gestagenen am Menschen im Kauffmann-Test und der wirksamkeit der Gestagene in der Klinik sehr gut korreliert.
*Ausführung:*
Infantilen weiblichen Neuseeländer-Kaninchen wurden nach einer 5tägigen Vorbehandlung mit 17β-Estradiolbenzoat vom 8. bis 12. Versuchstag diePrüfsubstanzen in öliger Lösung mittels Schlundsonde verabreicht.
Zur Bestimmung der Dienogestserumkonzentration erfolgte danach die Autopsie mit Blutentnahme.
Zur histologischen Untersuchung und Ermittlung des McPhail score wurde ein Uterushorn entnommen.
Die Abbildungen 1 bis 3 verdeutlichen die Ergebnisse der Prüfung.

Abbildung 1 zeigt die gestagene Aktivität der erfindungsgemäßen Verbindungen im Vergleich zu Dienogest am Kaninchenendometrium auf.

Es ist erkennbar, daß die meisten erfindungsgemäßen Verbindungen in äquimolaren Dosierungen eine stärkere Transformation des durch Estradiolbenzoat proliferierten Kaninchenendometriums bewirken als Dienogest - ersichtlich aus dem höheren McPhail score bei niedriger Dosierung.
Folglich sind die meisten Ester des Dienogest wesentlich wirksamer als Dienogest selbst.

Tabelle 1 zeigt die molare Bindungsaffinität (RBA) von Derivaten des Dienogest im Vergleich zu Dienogest zum Progesteron-Rezeptor des Kaninchen-Uterus-Cytosol.

| *Substanz* | *RBA [%]* |
|---|---|
| Progesteron | 100 |
| Dienogest | 10,5 |
| | |
| Butyrat | 0,26 |
| iso-Butyrat | 0,26 |
| Caproat | 0,27 |
| Caprylat | 0,34 |
| Pelargonat | 0,51 |
| Caprinat | 1,25 |
| Acetat | 0,31 |
| Propionat | 0,36 |
| Valerat | 0,25 |
| 3-Oxim | < 0,2 |
| 3-Oxim-sulfamat | < 0,1 |

Diese zusätzlichen Rezeptorbindungsstudien am Kaninchen-Uterus-Cytosol zeigen keine oder nur geringe Bindungsaffinitäten der erfindungsgemäßen Verbindungen.

Es ist abzuleiten, daß die Wirkform der erfindungsgemäßen Verbindungen Dienogest per se darstellt.

Überraschend ist nun, daß trotz wesentlich höherer gestagener Aktivität am Endometrium die Dienogest Dienogest-Wirkstoff-Spiegel nach oraler Applikation der erfindungsgemäßen Verbindungensignifikant niedriger sind als bei Applikation in gleicher Dosierung - ersichtlich aus Abbildung 2. Abbildung 2 zeigt den Dienogest-Spiegel (ng/ml) in Kaninchenserum nach oraler Gabe von Dienogest oder Dienogest-Estern in öliger Lösung (MW+/-SEM).

In Abbildung 3 ist der McPhail score als Parameter der gestagenen Wirkung in Beziehung zu den Dienogest-Serumspiegeln der Kaninchen aufgezeigt.
Auch hier wird bei den meisten erfindungsgemäßen Verbindungen verdeutlicht, daß mit niedrigeren Dienogest-Serumspiegeln ein höherer McPhail score , also eine stäörkere Wirkung am Endometrium erreicht wird.

Alle Ergebnisse beweisen die Möglichkeit einer wesentlich niedrigeren Dosierung der erfindungsgemäßen Verbindungen im Vergleich zu Dienogest und damit verbunden eine geringere Belastung des Organismus sowie eine Reduzierung der ohehin schon geringen Nebenwirkungsrate.

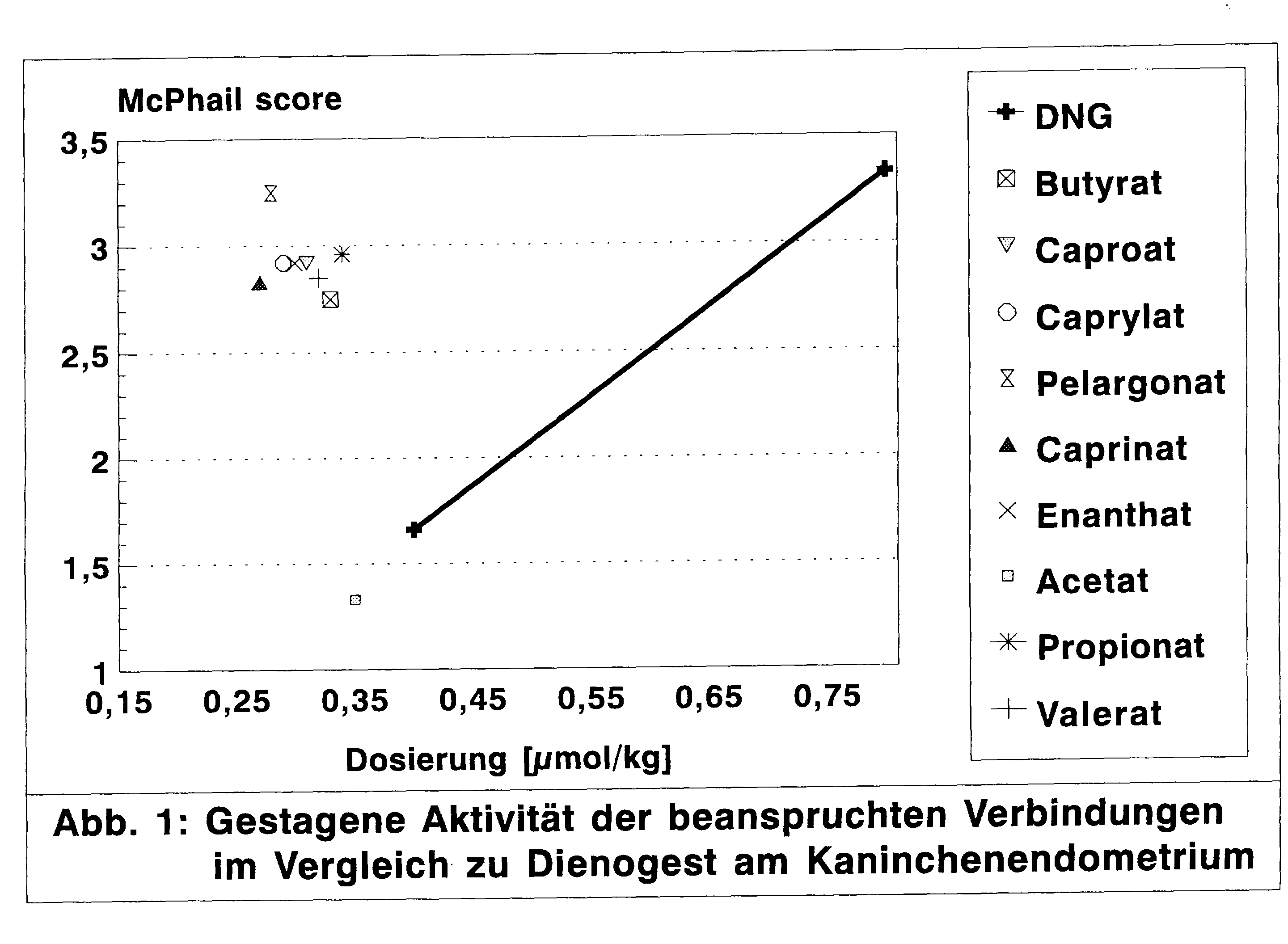

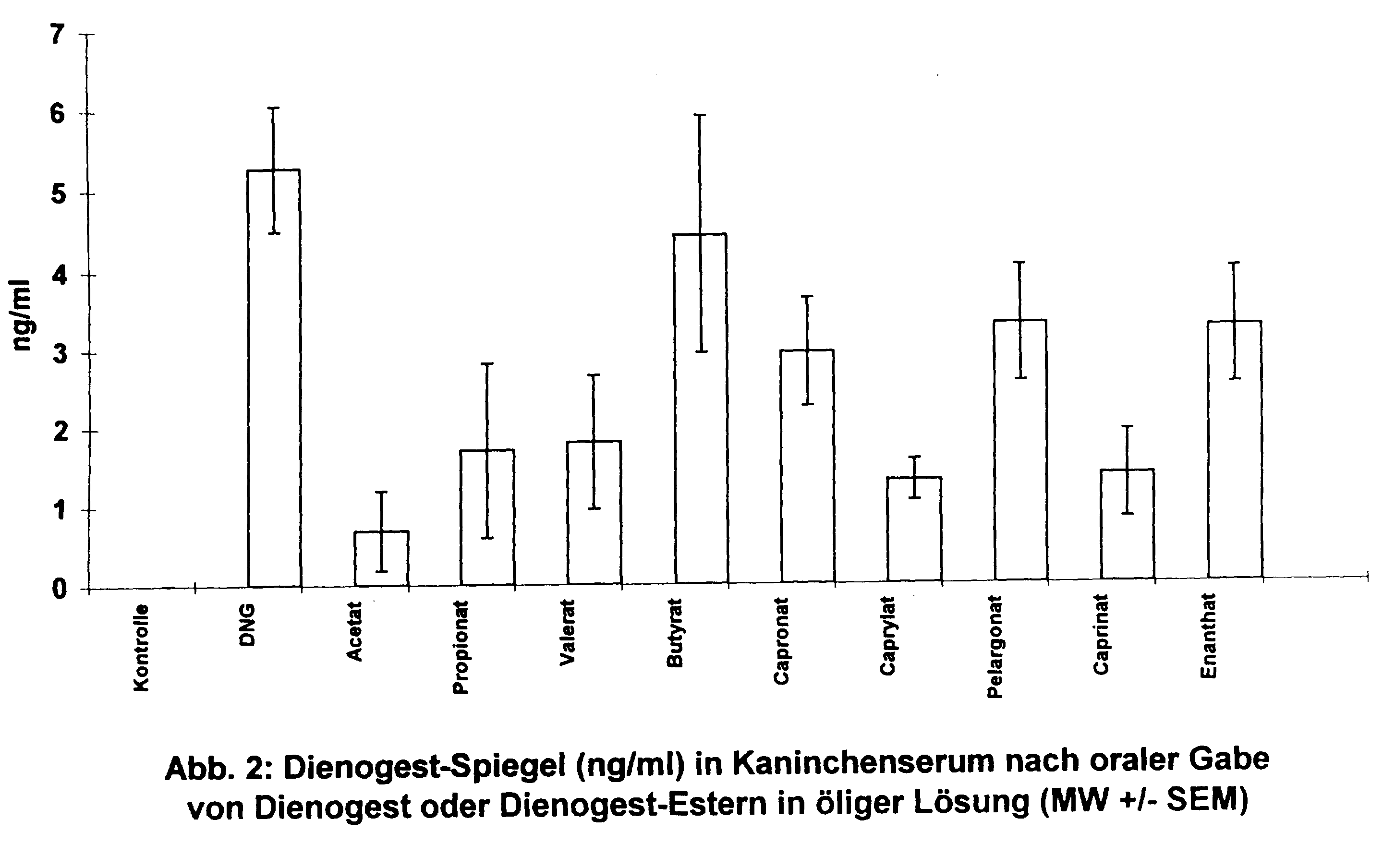

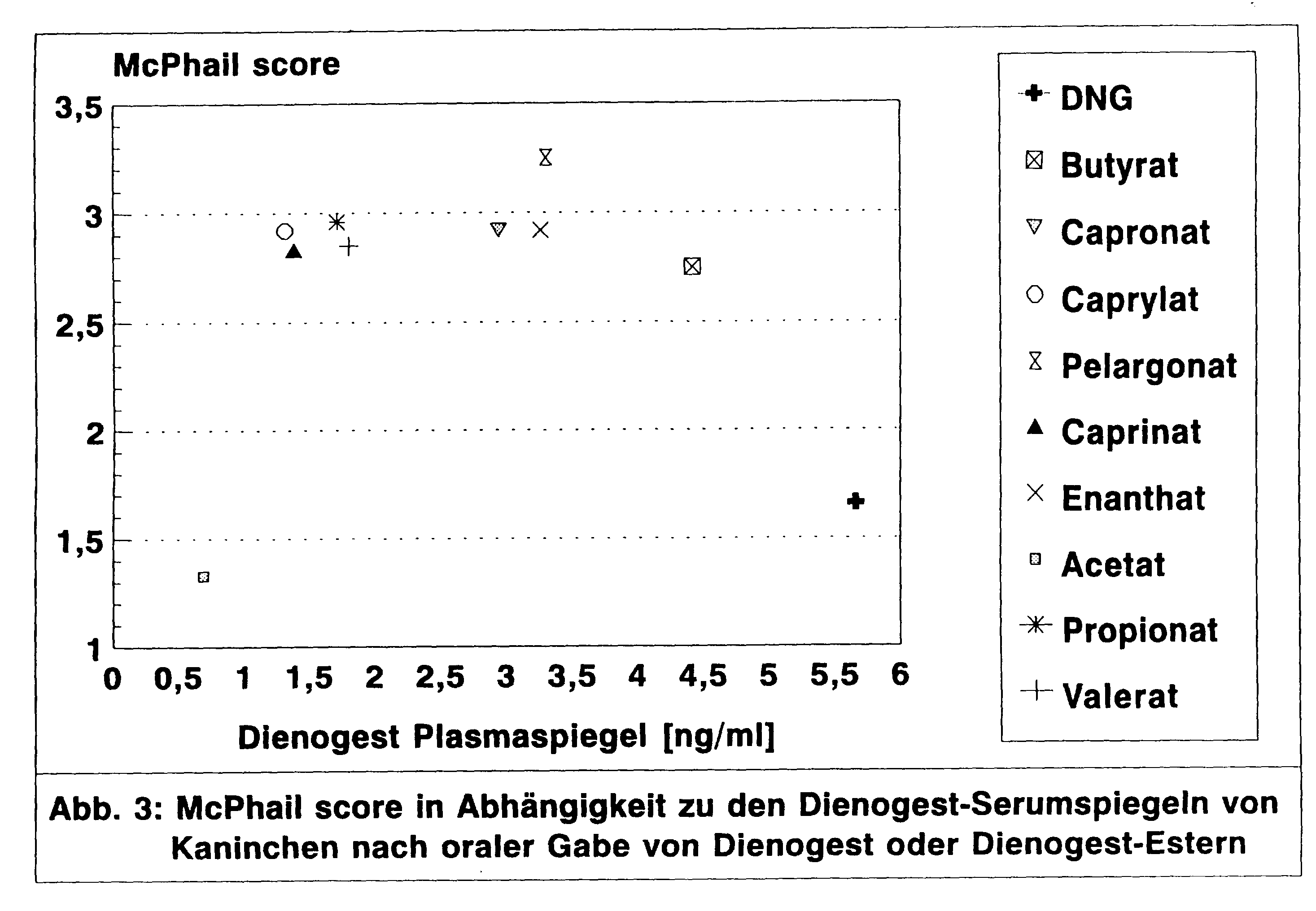

Aufgrund ihrer spezifischen hormonellen und antihormonalen Partialwirkungen können die neuen Substanzen zur Behandlung von Endokrinopathien und zur Reproduktionslenkung in der Human- und Veterinärmedizin eingesetzt werden.

Weiterhin können die erfindungsgemäßen Verbindungen wegen ihrer gestagenen Wirksamkeit allein oder in Kombination mit Estrogenen in Form von Mehrstufen- oder Kombinationspräparaten zur hormonalen Kontrazeption verwendet werden.

Vorzugsweise werden die gestagenen und estrogenen Wirkstoffkomponenten in Präparaten zur Kontrazeption zusammen oral appliziert. Als Estrogene kommen synthetische Estrogene, vorzugsweise aus der Gruppe Ethinylestradiol, Mestranol und biogene Estrogene, vorzugsweise aus der Gruppe Estradiol, Estron, Estriol in Betracht.

Die erfindungsgemäßen Verbindungen können auch in Präparaten zur Behandlung gynäkologischer Störungen und zur Substitutionstherapie eingesetzt werden. Durch ihr günstiges Wirksprofil sind die neuen Verbindungen zur Behandlung prämenstrueller Beschwerden geeignet.

Ferner können die pharmazeutischen Zusammensetzungen mit den erfindungsgemäßen Verbindungen zur Behandlung der Endometriose sowie zur Therapie gestagenabhängiger Tumore eingesetzt werden.

Die Arzneimittel der Erfindung werden mit üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in an sich bekannter Weise hergestellt.
Die bevorzugten Zubereitungen bestehen in einer Darreichungsform, die zur oralen Applikation geeignet ist.
Solche Darreichungsformen sind beispielsweise Tabletten, Filmtabletten, Dragees, Kapseln, Pillen, Pulver und Depotformen.

Es ist auch möglich die neuen Verbindungen in Suspensionen oder Lösungen einzuarbeiten.

Die Verwendung der erfindungsgemäßen Verbindungen als transdermale Systeme ist ebenfalls gegeben.

Die erfindungsgemäßen Verbindungen können auch als GestagenKomponente in Zusammensetzungen für die weibliche Fertilitätskontrolle eingesetzt werden, die sich durch die zusätzliche Verwendung eines kompetitiven Progesteronantagonisten auszeichnen( H.B: Croxatto et al, Female Contaception and Male Fertility Regulation, ed. By Runnebaum, Rabe & Kiesel, Vol.2, Advances in Gynecological and Obstetric Research Series, Parthenon Publishing Group, 1991, S.245).
Die zusätzlichen, kompetitiven Progesteronantagonisten können auch sequentiell appliziert werden.
Die erfindungsgemäßen Verbindungen stellen ferner Zwischenprodukte zur Synthese weiterer pharmakologisch hochwirksamer Steroidprodukte dar.
Die Herstellung der erfindungsgemäßen Verbindungen erfolgt in an sich bekannter Weise nach den Ansprüchen 3 bis 7.

Es werden Verbindungen der allgemeinen Formel II 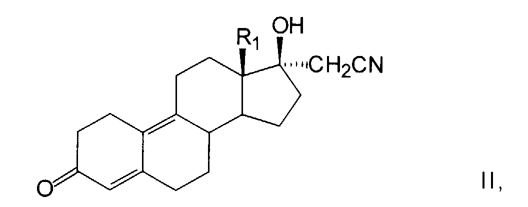 worin R₁ eine Alkylgruppe mit 1 bis 2 Kohlenstoffatomen bedeuten,in Gegenwart eines Amins mit einer Säure oder einem Säurederivat, wie beispielsweise Säureanhydrid, Säurehalogenid unter Zusatz eines Katalysators wie beispielsweise Dimethylaminopyridin zu den Esterderivaten der Formel (I) umgesetzt.
Vorteilhaft erfolgt die Durchführung der Reaktion unter Erwärmung und unter einer Schutzgasatmosphäre.

Die Isolierung der Reaktionsprodukte erfolgt durch Fällung in Eiswasser und Abtrennung der Festprodukte oder durch Extraktion mit geeigneten Lösungsmitteln.Die Anwendung inerter Lösungsmittel, die die Löslichkeit des Ausgangsmaterials wesentlich verbessern, ist nicht ausgeschlossen, insbesonders kommen hier solche Lösungsmittel in Betracht, die auch für einen Extraktionsprozeß geeignet sind, wie beispielsweise halogenierte Kohlenwasserstoffe, Essigsäureester, Ether wie tert-Butylmethylether u.a..

Eine weitere Synthesemöglichkeit besteht darin, daß man z.B. 17α-Cyanomethyl-17β-hydroxy-estra-4,9-dien-3-on (DIENOGEST) mit einem Alkenoylacylat, wie beispielsweise Isopropenylacetat zu Verbindungen der allgemeinen Formel III 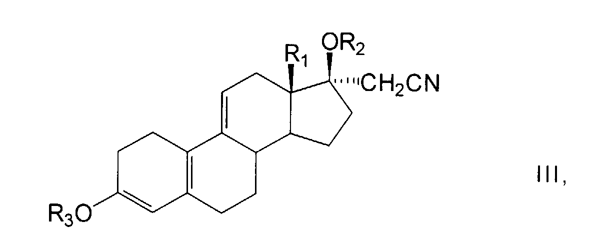 worin R₁ eine Alkylgruppe mit 1 bis 2 Kohlenstoffatomen, R₂ und R₃ ein Acylrest mit 1 bis 10 Kohlenstoffatomen bedeuten,verestert, diese isoliert und dann in einem wässerigen organischen Lösungsmittel in Gegenwart einer Säure, wie beispielsweise Salzsäure, Schwefelsäure, p-Toluolsulfonsäure bei erhöhten Reaktionstemperaturen zu den erfindungsgemäßen Verbindungen umsetzt und durch Fällung oder Extraktion isoliert.

Eine weitere Verfahrensvariante zur Herstellung von Verbindungen der allgemeinen Formel I, besteht darin, daß man Verbindungen der allgemeinen Formel IV, 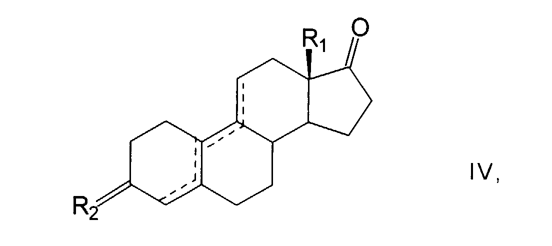 worin R₁ eine Alkylgruppe mit 1 bis 2 Kohlenstoffatomen, R₂ eine Dimethoxy-, Ethylendioxy-, 2,2-Dimethyl-1,3-propylendioxy gruppe bedeuten, in bekannter Weise durch Reaktion mit Lithium-acetonitril zum 17α-Cyanomethyl-derivat der allgemeinen Formel V umsetzt, oder die Verbindungen der allgemeinen Formel IV mit Trimethylsulfoniumjodid zum 17,17'-Spiroepoxyd umsetzt, welches dann durch Reaktion mit Alkalicyanid in Verbindungen der allgemeinen Formel V 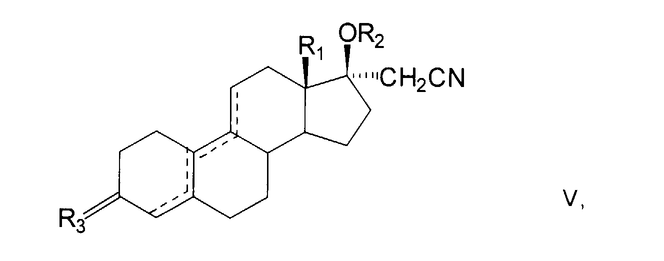 worin R₁ eine Alkylgruppe mit 1 bis 2 Kohlenstoffatomen, R₂ ein Wasserstoffatom oder ein Lithiumatom, R₃ eine Dimethoxy-, Ethylendioxy-, 2,2-Dimethyl-1,3-propylendioxygruppe darstellen, überführt wird, diese durch Veresterung in Verbindungen der Formel V 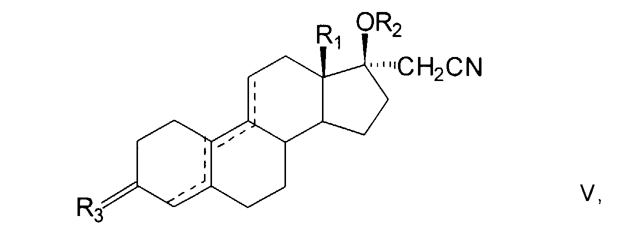 worin R₁ eine Alkylgruppe mit 1 bis 2 Kohlenstoffatomen, R₂ ein Acylrest mit 1 bis 10 Kohlenstoffatomen, R₃ eine Dimethoxy-, Ethylendioxy-, 2,2-Dimethyl-1,3-propylendioxygruppe bedeuten, umsetzt und nachfolgender Ketalspaltung in einem wässerig-organischen Lösungsmittel wie Essigsäure, Methanol, Aceton unter Zusatz eines Katalysators wie Salzsäure, Schwefelsäure, Salpetersäure, p-Toluolsulfonsäure, Brenztraubensäure gegebenenfalls unter Anwendung erhöhter Temperaturen in die erfindungsgemäßen Verbindungen überführt.

Weiterhin werden Verbindungen der allgemeinen Formel I 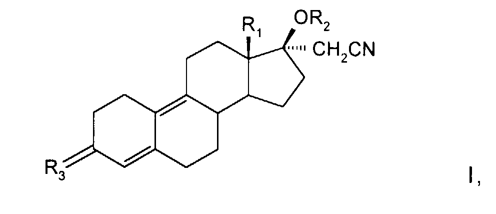 worin
R₁ eine Alkylgruppe mit 1 bis 2 Kohlenstoffatomen,
R₂ ein Acylrest mit 1 bis 10 Kohlenstoffatomen
und R₃ ein Sauerstoffatom darstellen, in an sich bekannter Weise mit Hydroxylammoniumchlorid in einem Amin, oder in einem inerten Lösungsmittel unter Zusatz säurebindender Mittel umgesetzt , und die Hydroxyiminosteroidderivate, in denen R₁ und R₂ die vorbezeichnete genannte Bedeutung besitzen und R₃ eine Hydroxyiminogruppe ist, isoliert
und / oder diese in an sich bekannter Weise mit einem Säurederivat, wie Carbonsäureanhydrid, Carbonsäurehalogenid, wie Carbonsäurechlorid oder Sulfamoylhalogenid, wie Sulfamoylchlorid in einem Amin, oder in einem inerten Lösungsmittel unter Zusatz eines Amins umsetzt und die Acyloxyiminoderivate, Sulfamoyloxyiminoderivate, in denen R₁ und R₂ die vorbezeichnete Bedeutung besitzen und R₃ ein Acyloxyimino- oder Sulfamoyloxyiminorest ist, isoliert
oder die Hydroxyiminoprodukte in an sich bekannter Weise mit Alkenen, wie Isobutylen, in inerten organischen Lösungsmitteln unter Zusatz von Katalysatoren wie Pyridinium-p-toluol-sulfonat, oder mit Trialkylsilylhalogeniden wie Triethylsilylbromid in inerten organischen Lösungsmitteln unter Zusatz eines Amins in Alkyloxyiminoderivate bzw. Trialkylsilyloxyiminoderivate überführt, in denen R₁ und R₂ die oben genannte Bedeutung besitzen und R₃ ein Alkyloxyimino- oder Trialkylsilyloxyiminorest ist.

Eine weitere Verfahrensvariante zur Herstellung von Verbindungen der allgemeinen Formel I besteht darin, daß Verbindungen der allgemeinen Formel VI, 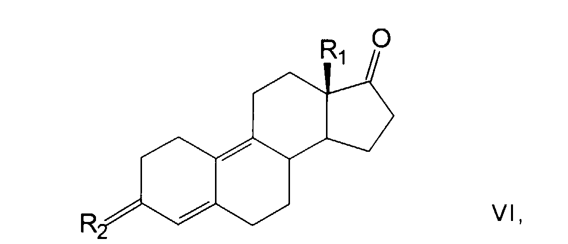 worin R₁ eine Alkylgruppe mit 1 bis 2 Kohlenstoffatomen und R₂ eine Hydroxyiminogruppe, in an sich bekannter Weise mit Lithiumacetonitril oder mit Trimethylsulfoniumjodid und anschließender Behandlung mit einem Alkalicyanid zu Verbindungen der allgemeinen Formel I, worin R₁ eine Alkylgruppe mit 1 bis 2 Kohlenstoffatomen, R₂ ein Wasserstoffatom und R₃ eine Hydroxyiminogruppe ist, umsetzt und diese in an sich bekannter Weise mit einem Säurederivat, wie Carbonsäureanhydrid oder Carbonsäurehalogenid in einem Amin, oder in einem inerten Lösungsmittel unter Zusatz eines Amins umsetzt und die Acylderivate in denen R₁ die vorbezeichnete Bedeutung besitzt und R₂ ein C₁₋₁₀ Acyl- und R₃ ein C₁₋₁₀ Acyloyloxyiminorest ist, isoliert.

Die pharmazeutischen Zubereitungen, die mindestens ein 17α-Cyanomethylestra-4,9-dienderivat nach Anspruch 1 und 2 enthalten, umfassen gegebenenfalls pharmazeutisch verträgliche Hilfs- und Trägerstoffe.

Die Herstellung der erfindungsgemäßen Verbindungen der allgemeinen Formel I soll an den nachfolgenden Beispielen näher erläutert, jedoch nicht näher eingeschränkt werden.

### Beispiel 1

### 17α-Cyanomethyl-3-hydroxyiminoestra-4,9-dien -17β-yl-acetat

5,0 g 17α-Cyanomethyl-estra-4,9-dien -17β-yl-acetat werden in 25 ml Pyridin mit 2,5 g Hydroxylammoniumhydrochlorid versetzt. Nach einer Stunde Reaktionszeit bei 35°C gibt man das Reaktionsgemisch unter Rühren in 1 l Eiswasser, saugt das Fällungsprodukt ab, wäscht mit Wasser und trocknet.
Das Rohprodukt wird aus Methanol umkristallisiert, dann in CH₂Cl₂ über Kieselgel chromatographiert und nochmals aus Methanol kristallisiert.
Ausbeute :2,3 g

### Beispiel 2

### 17α-Cyanomethyl-3-hydroxyiminoestra-4,9-dien -17β-yl-propionat

3,5 g 17α-Cyanomethylestra-4,9-dien-17β-yl-propionat werden in 17,5 ml Pyridin mit 875 mg Hydroxylammoniumchlorid versetzt. Vollständige Umsetzung war nach 2,5 Stunden Reaktionszeit bei 35°C er reicht. Das Reaktionsgemisch wird unter Rühren in 750 ml Eiswasser gefällt, abgesaugt und gewaschen.
Das Rohprodukt wird in Methylenchlorid / Methanol zur Kristallisation gebracht, das Kristallisat abgesaugt, mit Methanol gewaschen und getrocknet.
Ausbeute :3,08 g

### Beispiel 3

### 3-Acetyloxyimino-17α-cyanomethylestra-4,9-dien-17β-yl-acetat

2 g 17α-Cyanomethyl-3-hydroxyiminoestra-4,9-dien-17β-yl-acetat werden in 8 ml Pyridin mit 8 ml Acetanhydrid und 100 mg Dimethylaminopyridin versetzt. Vollständige Umsetzung war nach 1 Stunde bei 40°C erreicht.
Die Isolierung des Produktes erfolgte durch Fällung in Eiswasser und Kristallisation aus Methanol.

### Beispiel 4

### 17α-Cyanomethyl-3-valeroyloxyiminoestra-4,9-dien-17β-yl-acetat

1,5 g 17α-Cyanomethyl-3-hydroxyiminoestra-4,9-dien-17β-yl-acetat wer den in 6 ml Pyridin und 6 ml Valeriansäureanhydrid unter Zusatz von 75 mg Dimethylaminopyridin bei 45°C innerhalb 1 Stunde vollständig umgesetzt.
Anschließend gibt man in Eiswasser, extrahiert mit Methylenchlorid wäscht die Lösumung säurefrei und engt ein zum Festprodukt.

### Beispiel 5

### 17α-Cyanomethyl-3-phenylcarbamoyloxyiminoestra-4,9-dien-17β-ylacetat

1,5 g 17α-Cyanomethyl-3-hydroxyiminoestra-4,9-dien-17β-yl-acetat wer den in 40 ml Toluol, 2 ml Triethylaminunter Rühren bei Raumtemperatur mit von 0,6 ml Phenylisocyanat versetzt.
Nach vollständiger Umsetzung wird das Gemisch mit 5 ml Methanol und dann mit 50 ml Wasser versetzt, mit Toluol extrahiert, die Lösung mit Wasser gewaschen, eingeengt und der Rückstand aus Methylenchlorid / Methanol kristallisiert.
Ausbeute : 1,6 g
Fp : 170 - 188°C

### Beispiel 6

### 17α-Cyanomethyl-3-methoxycarbonyloxyiminoestra-4,9-dien-17β-ylacetat

2 g 17α-Cyanomethyl-3-hydroxyiminoestra-4,9-dien-17β-yl-acetat werden in 10 ml Pyridin gelöst, die Lösung auf -5°C abgekühlt und dann unter Rühren mit einer Lösung von 0,82 ml Methylchloroformiat in 20 ml Toluol versetzt. Nach 1 Stunde Reaktion bei Raumtemperatur war vollständige Umsetzung erfolgt.
Das Reaktionsgemisch wurde durch Zusatz von 5 ml Methanol und dann mit 50 ml Wasser zersetzt, mit Toluol extrahiert, der Extrakt mit Wasser gewaschen, eingeengt und der erhaltene Rückstand aus Methanol umkristallisiert.
Ausbeute : 1,42 g
Fp : 126 - 132°C

### Beispiel 7

### 3-Benzoyloxy-17α-cyanomethylestra-4,9-dien-17β-yl-acetat

2 g 17α-Cyanomethyl-3-hydroxyiminoestra-4,9-dien-17β-yl-acetat werden in 10 ml Pyridin gelöst und bei Raumtemperatur unter Rühren mit 1,2 ml Benzoylchlorid versetzt, wobei ein Temperaturanstieg bis auf 35°C erfolgte. Nach 15 Minuten Reaktionszeit war die vollständige Umsetzung erfolgt.
Das Reaktionsgemisch wurde in Eiswasser eingerührt, mit Methylenchlorid extrahiert, das Fällungsprodukt wird abgesaugt, mit Wasser gewaschen und dann aus Methylenchlorid / Methanol umkristallisiert.
Ausbeute : 2,24 g Fp : 190 - 215°C

### Beispiel 8

### 17α-Cyanomethyl-3-N,N-dimethylcarbamoyloxyiminoestra-4,9-dien-17β-yl-acetat

2 g 17α-Cyanomethyl-3-hydroxyiminoestra-4,9-dien -17β-yl-acetat werden in 10 ml Pyridin gelöst, die Lösung auf -6°C abgekühlt und unter Rühren mit einer Lösung von 0,75 ml N,N-Dimethylcarbamoylchlorid in 20 ml Toluol versetzt. Nach 5 Stunden Reaktionszeit bei 50°C war vollständige Umsetzung erfolgt, das Reaktionsgemisch wurde duch Zusatz von 5 ml Methanol und 25 ml Wasser zersetzt, die Toluollösung abgetrennt, eingeengt, in Methylenchlorid aufgenommen, mit Wasser gewaschen, eingeengt und der Rückstand aus Methanol kristallisiert.
Ausbeute : 1,35 g
Fp: 97 110°C

### Beispiel 9

### 17α-Cyanomethylestra-4,9-dien-3-on-17β-yl-acetat

10 g 17α-Cyanomethylestra4,9-dien-3-on-17β ol, 50 ml Pyridin, 50 ml Acetanhydrid und 500 mg Dimethylaminopyridin werden in einer Inertgasatmosphere unter Rühren bis auf 80°C erwärmt.
Nach vollständiger Umsetzung (DC-Kontrolle ) wird das Reaktionsgemisch abgekühlt und unter Rühren in Eiswasser gefällt.
Das Fällungsprodukt wird abgesaugt, gut mit Wasser gewaschen, getrocknet, dann in Methylenchlorid gelöst, die Lösung mit Methanol versetzt und unter Vakuum bis zur beginnenden Kristallisation eingeengt. Das Kristallisat wird abgesaugt, mit kaltem Methanol gewaschen und getrocknet.

### Beispiel 10

### 17α-Cyanomethylestra-4,9-dien-3-on-17β-yl-propionat

10 g 17α-Cyanomethylestra4,9-dien-3-on-17β ol, 50 ml Pyridin, 50 ml Propionsäureanhydrid und 500 mg Dimethylaminopyridin werden in einer Inertgasatmosphere unter Rühren bis auf 80°C erwärmt. Nach vollständiger Umsetzung (DC-Kontrolle ) wird das Reaktionsgemisch abgekühlt und unter Rühren in Eiswasser gefällt.
Das Fällungsprodukt wird abgesaugt, gut mit Wasser gewaschen, getrocknet, dann in Methylenchlorid gelöst, die Lösung mit Methanol versetzt und unter Vakuum bis zur beginnenden Kristallisation eingeengt. Das Kristallisat wird abgesaugt, mit kaltem Methanol gewaschen und getrocknet.

### Beispiel 11

### 1 7α-Cyanomethylestra-4,9-dien-3-on-17β-yl-valerat

5 g 17α-Cyanomethylestra-4,9-dien-3-on-17β-ol, 20 ml Pyridin, 20 ml Valeriansäureanhydrid und 250 mg 4-(Dimethylamino)-pyridin werden unter Rühren bis auf 96°C erwärmt, 5 Stdn. bei dieser Temperatur gehalten, dann abgekühlt und unter Rühren in 1 Eiswasser gefällt. Das Festprodukt wird abgesaugt,gewaschen und getrocknet.
Ausbeute / Rohprodukt: 6,3 g
Umkristallisation aus Methanol ergibt 4,8 g krist. Produkt,
Chromatogr. der CH₂Cl₂-Lösung über Kieselgel und Kristallisation aus Methanol ergibt das reine Produkt.
Ausbeute: 2,0 g
FP: 152 - 155°C

## Patentansprüche

1. 17α-Cyanomethylestra-4,9-dien-derivate der allgemeinen Formel I 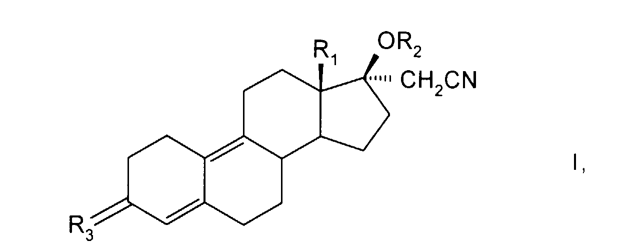 worin
R₁ eine Alkylgruppe mit 1 bis 2 Kohlenstoffatomen,
R₂ ein Acylrest mit 1 bis 10 Kohlenstoffatomen,
R₃ ein Sauerstoffatom oder eine R-O-N-Gruppierung,
wobei
R ein Wasserstoffatom, ein Acylrest mit 1 bis 10 Kohlenstoffatomen, ein Alkylrest mit 1 bis 10 Kohlenstoffatomen, ein Sulfamoylrest oder Trialkylsilylrest darstellen,
bedeuten.

2. 17α-Cyanomethylestra-4,9-dien-derivate nach Anspruch 1, nämlich
17α-Cyanomethylestra-4,9-dien-3-on-17β-yl-acetat,
17α-Cyanomethylestra-4,9-dien-3-on-17β-yl-propionat,
17α-Cyanomethylestra-4,9-dien-3-on-17β-yl-butyrat,
17α-Cyanomethylestra-4,9-dien-3-on-17β-yl-iso-butyrat,
17α-Cyanomethylestra-4,9-dien-3-on-17β-yl-valerat,
17α-Cyanomethylestra-4,9-dien-3-on-17β-yl-iso-valerat,
17α-Cyanomethylestra-4,9-dien-3-on-17β-yl-decanoat,
17α-Cyanomethylestra-4,9-dien-3-on-17β-yl-cyclohexanoat,
17α-Cyanomethylestra-4,9-dien-3-on-17β-yl-benzoat,
17α-Cyanomethyl-3-hydroxyiminoestra-4,9-dien-17β-yl-acetat,
17α-Cyanomethyl-3-hydroxyiminoestra-4,9-dien-17β-yl-propionat,
17α-Cyanomethyl-3-hydroxyiminoestra-4,9-dien-17β-yl-butyrat,
17α-Cyanomethyl-3-hydroxyiminoestra-4,9-dien-17β-yl-valerat,
17α-Cyanomethyl-3-hydroxyiminoestra-4,9-dien-17β-yl-cyclopentanoat,
17α-Cyanomethyl-3-hydroxyiminoestra-4,9-dien-17β-yl-benzoat,
17α-Cyanomethyl-3-hydroxyiminoestra-4,9-dien-17β-yl-decanoat,
3-Acetoxyimino-17α-cyanomethylestra-4,9-dien-17β-yl-acetat,
3-Acetoxyimino-17α-cyanomethylestra-4,9-dien-17β-yl-valerat,
3-Acetoxyimino-17α-cyanomethylestra-4,9-dien-17β-yl-benzoat,
17α-Cyanomethyl-3-propionyloxyimino-estra-4,9-dien-17β-ylacetat,
3-Benzoyloxyimino -17α-cyanomethylestra-4,9-dien-17β-yl-acetat,
3-Benzoyloxyimino-17α-cyanomethylestra-4,9-dien-17β-yl-benzoat,
3-tert-Butoxyimino-17α-cyanomethyl-estra-4,9-dien-17β-yl-acetat,
3-tert-Butoxyimino -17α-cyanomethyl-estra-4,9-dien-17β-yl-benzoat,
17α-Cyanomethyl-3-( N,N-diethylsulfamoyloxy )-imino-estra-4,9-dien-17β-yl-acetat,
17α-Cyanomethyl-3-( N-acetylsulfamoyloxy )-imino-estra-4,9-dien-17β-yl-valerianat.

3. Verfahren zur Herstellung von 17α-Cyanomethylestra-4,9-dien-derivaten
nach den Ansprüchen 1 und 2,
dadurch gekennzeichnet,
daß man Verbindungen der allgemeinen Formel II, 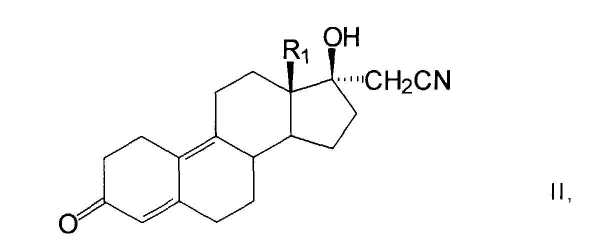 worin
R₁ eine Alkylgruppe mit 1 bis 2 Kohlenstoffatomen darstellt,
in an sich bekannter Weise mit einem Säureanhydrid, Säurehalogenid in Gegenwart eines Amins unter Zusatz eines Katalysators zum Produkt des Anspruchs 1 verestert.

4. Verfahren zur Herstellung von
17α-Cyanomethylestra-4,9-dien-derivaten
nach den Ansprüchen 1 und 2,
dadurch gekennzeichnet,
daß man Verbindungen der allgemeinen Formel II,
worin
R₁ die im Anspruch 3 genannten Bedeutungen aufweisen in an sich bekannter Weise mit einem Alkenoyl-Acylderivat zu Verbindungen der allgemeinen Formel III, 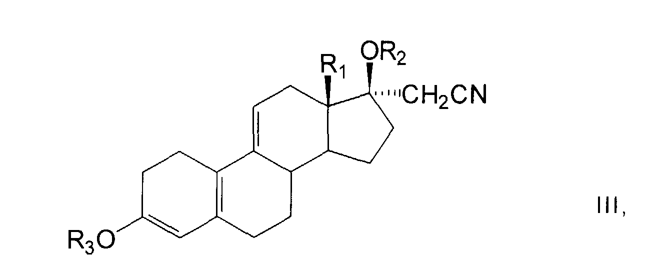 worin
R₁ eine Alkylgruppe mit 1 bis 2 Kohlenstoffatomen
R_{2,} R₃ ein Acylrest mit 1 bis 10 Kohlenstoffatomen
bedeuten,
verestert
und in einem wässerig-organischen Lösungsmittel unter Zusatz eines Katalysators einer Enolesterspaltung unterwirft.

5. Verfahren zur Herstellung von
17α-Cyanomethylestra-4,9-dien-derivaten
nach den Ansprüchen 1 und 2,
dadurch gekennzeichnet,
daß man Verbindungen der allgemeinen Formel IV, 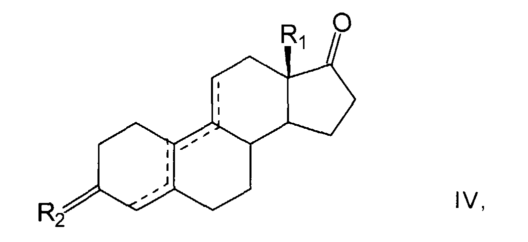 worin
R₁ eine Alkylgruppe mit 1 bis 2 Kohlenstoffatomen,
R₂ eine Dimethoxy-, Ethylendioxy-,
2,2-Dimethyl-1,3-propylendioxygruppe
bedeuten,
in an sich bekannter Weise zum 17α-Cyanomethylderivat der allgemeinen Formel V, worin
R₁ eine Alkylgruppe mit 1 bis 2 Kohlenstoffatomen,
R₂ ein Wasserstoffatom, ein Lithiumatom,
R₃ eine Dimethoxy-,Ethylendioxy-, 2,2-Dimethyl-1,3-propylendioxygruppe darstellen,
umsetzt,
in an sich bekannter Weise mit einem Säureanhydrid oder Säurehalogenid in Gegenwart eines Amins unter Zusatz eines Katalysators
und analog den Ansprüchen 3
zu den Verbindungen der allgemeinen Formel V 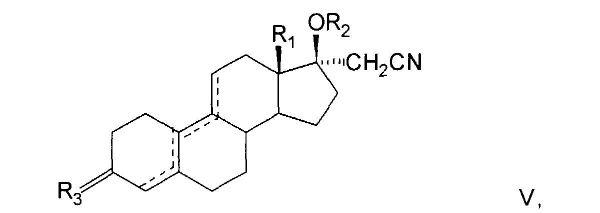 worin
R₁ eine Alkylgruppe mit 1 bis 2 Kohlenstoffatomen
R₂ ein Acylrest mit 1 bis 10 Kohlenstoffatomen
R₃ eine Dimethoxy-, Ethylendioxy-,
2,2-Dimethyl-1,3-propylendioxygruppe
bedeuten,
verestert,
einer Ketalspaltung in einem wässrigorganischen Lösungsmittel unter Zusatz eines Katalysators unterwirft.

6. Verfahren zur Herstellung von 17α-Cyanomethylestra-4,9-dien-derivaten
nach den Ansprüchen 1 und 2,
dadurch gekennzeichnet,
daß man Verbindungen der allgemeinen Formel I, 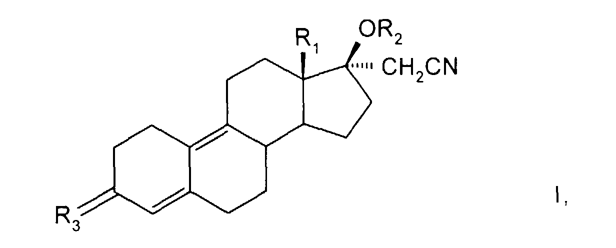 worin
R₁ eine Alkylgruppe mit 1 bis 2 Kohlenstoffatomen,
R₂ ein Acylrest mit 1 bis 10 Kohlenstoffatomen,
R₃ ein Sauerstoffatom
darstellen,
in an sich bekannter Weise mit Hydroxylammoniumchlorid in einem Amin oder in einem inerten Lösungsmittel unter Zusatz säurebindender Mittel umsetzt
und die Hydroxyiminosteroidderivate,
worin
R₁ und R₂ die vorbezeichnete Bedeutung besitzen
R₃ eine Hydroxyiminogruppe ist,
isoliert
und / oder diese in an sich bekannter Weise mit einem Säurederivat in einem Amin oder in einem inerten Lösungsmittel unter Zusatz eines Amins umsetzt
und die Acyloxyiminoderivate, Sulfamoyloxyiminoderivate,
worin
R₁ und R₂ die oben vorbezeichnete Bedeutung besitzen und
R₃ ein Acyloxyimino- oder Sulfamoyloxyiminorest ist,
isoliert
oder die Hydroxyiminoprodukte in an sich bekannter Weise mit Alkenen in inerten organischen Lösungsmitteln unter Zusatz von Katalysatoren
oder mit Trialkylsilylhalogeniden in inerten organischen Lösungsmitteln unter Zusatz eines Amins in Alkyloxyiminoderivate bzw. Trialkylsilyloxyiminoderivate überführt,
worin
R₁ und R₂ die vorbezeichnete Bedeutung besitzen,
R₃ ein Alkoxyimino- oder Trialkylsilyloxyiminorest ist.

7. Verfahren zur Herstellung 17α-Cyanomethylestra-4,9-dien-derivaten
nach den Ansprüchen 1 und 2,
dadurch gekennzeichnet,
daß man Verbindungen der allgemeinen Formel VI 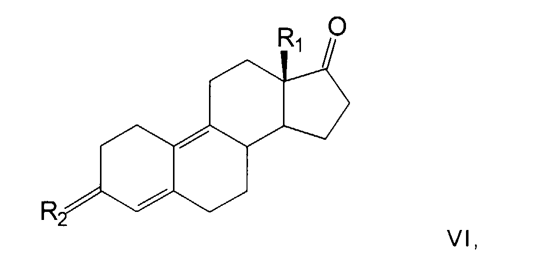 worin
R₁ eine Alkylgruppe mit 1 bis 2 Kohlenstoffatomen,
R₂ eine Hydroxyiminogruppe ist
in bekannter Weise mit Lithiumacetonitril
oder
mit Trimethylsulfoniumjodid und anschließender Behandlung mit einem Alkalicyanid zu Verbindungen der allgemeinen Formel I, 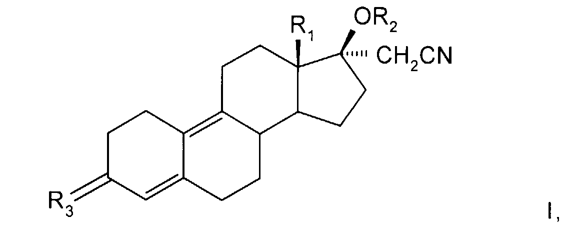 worin
R₁ eine Alkylgruppe mit 1 bis 2 Kohlenstoffatomen,
R₂ ein Wasserstoffatom und
R₃ eine Hydroxyiminogruppe ist,
umsetzt
und diese in an sich bekannter Weise mit einem Säurederivat in einem Amin oder in einem inerten Lösungsmittel unter Zusatz eines Amins umsetzt
und die Acylderivate,
worin
R₁ die oben vorbezeichnete Bedeutung besitzt und
R₂ ein C₁₋₁₀ Acyl und
R₃ ein C₁₋₁₀ Acyloxyimino ist,
isoliert.

8. Pharmazeutische Zusammensetzungen
enthaltend mindestens ein 17α-Cyanomethylestra-4,9-dien-derivat nach Anspruch 1 oder 2 gegebenenfalls zusammen mit pharmazeutisch verträglichen Hilfs-und Trägerstoffen.

## Claims

1. 17α-Cyanomethyloestra-4,9-diene derivatives of the general formula I 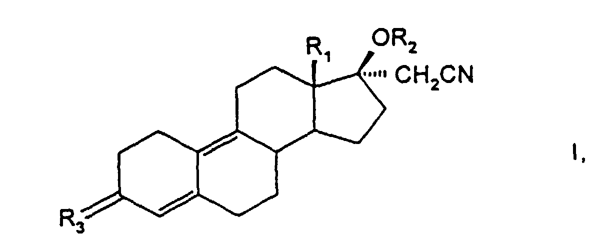 in which
R₁ signifies an alkyl group with from 1 to 2 carbon atoms,
R₂ signifies an acyl residue with from 1 to 10 carbon atoms,
R₃ signifies an oxygen atom or an R-O-N grouping,
wherein
R represents a hydrogen atom, an acyl residue with from 1 to 10 carbon atoms, an alkyl residue with from 1 to 10 carbon atoms, a sulphamoyl residue or a trialkylsilyl residue.

2. 17α-Cyanomethyloestra-4,9-diene derivatives according to claim 1, namely
17α-cyanomethyloestra-4,9-dien-3-on-17β-yl acetate,
17α-cyanomethyloestra-4,9-dien-3-on-17β-yl propionate,
17α-cyanomethyloestra-4,9-dien-3-on-17β-yl butyrate,
17α-cyanomethyloestra-4,9-dien-3-on-17β-yl isobutyrate,
17α-cyanomethyloestra-4,9-dien-3-on-17β-yl valerate,
17α-cyanomethyloestra-4,9-dien-3-on-17β-yl isovalerate,
17α-cyanomethyloestra-4,9-dien-3-on-17β-yl decanoate,
17α-cyanomethyloestra-4,9-dien-3-on-17β-yl cyclohexanoate,
17α-cyanomethyloestra-4,9-dien-3-on-17β-yl benzoate,
17α-cyanomethyl-3-hydroxyimino-oestra-4,9-dien-17β-yl acetate,
17α-cyanomethyl-3-hydroxyimino-oestra-4,9-dien-17β-yl propionate,
17α-cyanomethyl-3-hydroxyimino-oestra-4,9-dien-17β-yl butyrate,
17α-cyanomethyl-3-hydroxyimino-oestra-4,9-dien-17β-yl valerate,
17α-cyanomethyl-3-hydroxyimino-oestra-4,9-dien-17β-yl cyclopentanoate,
17α-cyanomethyl-3-hydroxyimino-oestra-4,9-dien-17β-yl benzoate,
17α-cyanomethyl-3-hydroxyimino-oestra-4,9-dien-17β-yl decanoate,
3-acetoxyimino-17α-cyanomethyloestra-4,9-dien-17β-y1 acetate,
3-acetoxyimino-17α-cyanomethyloestra-4,9-dien-17β-y1 valerate,
3-acetoxyimino-17α-cyanomethyloestra-4,9-dien-17β-y1 benzoate,
17α-cyanomethyl-3-propionyloxyimino-oestra-4,9-dien-17β-yl acetate
3-benzoyloxyimino-17α-cyanomethyloestra-4,9-dien-17β-yl acetate,
3-benzoyloxyimino-17α-cyanomethyloestra-4,9-dien-17β-yl benzoate,
3-tert-butoxyimino-17α-cyanomethyl-oestra-4,9-dien-17β-yl acetate,
3-tert-butoxyimino-17α-cyanomethyl-oestra-4,9-dien-17β-yl benzoate,
17α-cyanomethyl-3-(N,N-diethylsulphamoyloxy)-imino-oestra-4,9-dien-17β-yl acetate,
17α-cyanomethyl-3-(N-acetylsulphamoyloxy)-imino-oestra-4,9-dien-17β-yl valerate.

3. A method of producing 17α-cyanomethyloestra-4,9-diene derivatives according to claims 1 and 2, characterised in that compounds of the general formula II 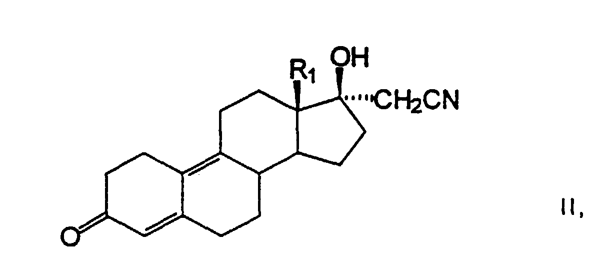 in which
R₁ represents an alkyl group with from 1 to 2 carbon atoms,
are esterified in a manner known per se with an acid anhydride or acid halide in the presence of an amine and with the addition of a catalyst to form the product of claim 1.

4. A method of producing 17α-cyanomethyloestra-4,9-diene derivatives according to claims 1 and 2, characterised in that compounds of the general formula II,
in which
R₁ has the meanings given in claim 3,
are esterified in a manner known per se with an alkenoyl/acyl derivative to form compounds of the general formula III, 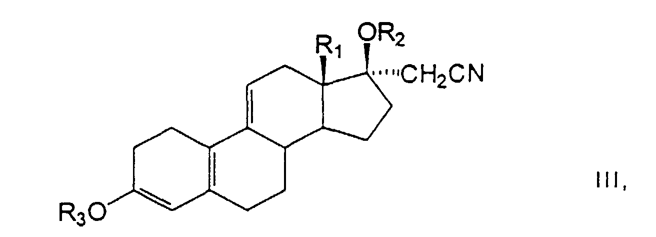 in which
R₁ signifies an alkyl group with from 1 to 2 carbon atoms and
R₂ and R₃ signify an acyl residue with from 1 to 10 carbon atoms,
and subjected to enol ester cleavage in an aqueous organic solvent with the addition of a catalyst.

5. A method of producing 17α-cyanomethyloestra-4,9-diene derivatives according to claims 1 and 2, characterised in that compounds of the general formula IV 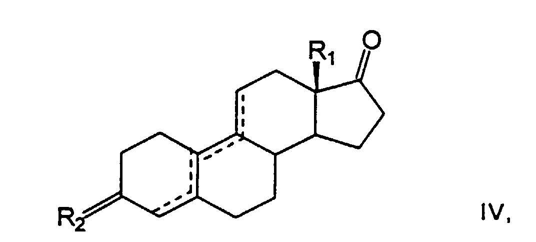 in which
R₁ signifies an alkyl group with from 1 to 2 carbon atoms and
R₂ signifies a dimethoxy, ethylenedioxy or 2,2-dimethyl-1,3-propylenedioxy group,
are reacted in a manner known per se to form a 17α-cyanomethyl derivative of the general formula V,
in which
R₁ represents an alkyl group with from 1 to 2 carbon atoms,
R₂ represents a hydrogen atom or a lithium atom and R₃ represents a dimethoxy, ethylenedioxy or 2,2-dimethyl-1,3-propylenedioxy group,
esterified in a manner known per se and as in claim 3 with an acid anhydride or an acid halide in the presence of an amine and with the addition of a catalyst to form the compounds of the general formula V 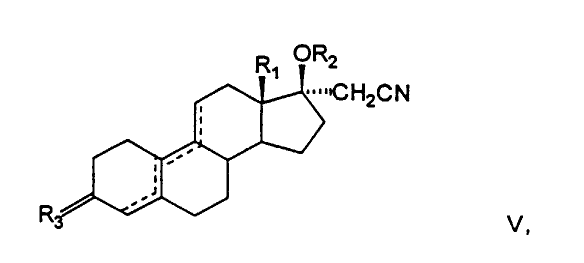 in which
R₁ signifies an alkyl group with from 1 to 2 carbon atoms,
R₂ signifies an acyl residue with from 1 to 10 carbon atoms and
R₃ signifies a dimethoxy, ethylenedioxy or 2,2-dimethyl-1,3-propylenedioxy group,
and subjected to ketal cleavage in an aqueous organic solvent with the addition of a catalyst.

6. A method of producing 17α-cyanomethyloestra-4,9-diene derivatives according to claims 1 and 2, characterised in that compounds of the general formula I 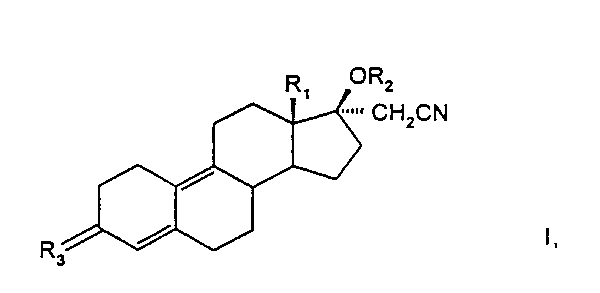 in which
R₁ represents an alkyl group with from 1 to 2 carbon atoms,
R₂ represents an acyl residue with from 1to 10 carbon atoms and
R₃ represents an oxygen atom,
are reacted in a manner known per se with hydroxyl ammonium chloride in an amine or in an inert solvent with the addition of an acid-binding agent
and the hydroxyiminosteroid derivatives,
in which
R₁ and R₂ have the above-defined meanings and
R₃ is a hydroxyimino group,
are isolated
and/or the latter are reacted in a manner known per se with an acid derivative in an amine or in an inert solvent with the addition of an amine
and the acyloxyimino derivatives and sulphamoyloxyimino derivatives,
in which
R₁ and R₂ have the above-defined meanings and
R₃ is an acyloxyimino or sulphamoyloxyimino residue,
are isolated
or the hydroxyimino products are converted, in a manner known per se with alkenes in inert organic solvents with the addition of catalysts
or with trialkylsilyl halides in inert organic solvents with the addition of an amine, respectively into alkyloxyimino derivatives or trialkylsilyloxyimino derivatives, in which
R₁ and R₂ have the above-defined meanings and
R₃ is an alkoxyimino or trialkylsilyloxyimino residue.

7. A method of producing 17α-cyanomethyloestra-4,9-diene derivatives according to claims 1 and 2, characterised in that compounds of the general formula VI 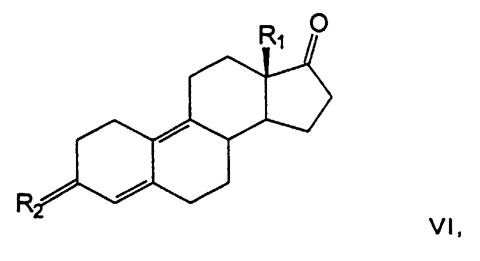 in which
R₁ is an alkyl group with from 1 to 2 carbon atoms and
R₂ is a hydroxyimino group,
are reacted in a known way with lithium acetonitrile or
with trimethylsulphonium iodide and subsequent treatment with an alkali cyanide to form compounds of the general formula I, 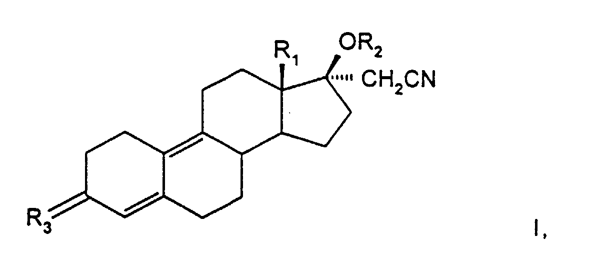 in which
R₁ is an alkyl group with from 1 to 2 carbon atoms,
R₂ is a hydrogen atom and
R₃ is a hydroxyimino group,
and the latter are reacted in a manner known per se with an acid derivative in an amine or in an inert solvent with the addition of an amine
and the acyl derivatives,
in which
R₁ has the above-defined meaning and
R₂ is a C₁₋₁₀ acyl and
R₃ is a C₁₋₁₀ acyloxyimino,
are isolated.

8. Pharmaceutical compositions containing at least one 17α-cyanomethyloestra-4,9-diene derivative according to claim 1 or claim 2, optionally together with pharmaceutically acceptable auxiliary substances and carriers.

## Revendications

1. Dérivés de 17α-cyanométhylestra-4,9-diène de la formule générale I 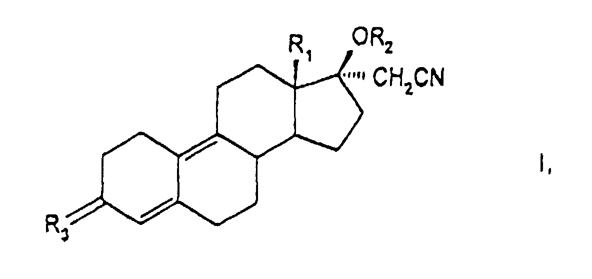 où
R₁ représente un groupe alkyle ayant 1 à 2 atomes de carbone,
R_{2,} un résidu acyle de 1 à 10 atomes de carbone
R_{3,} un atome d'oxygène ou un groupe R-O-N
où R représente un atome d'hydrogène, un résidu acyle avec 1 à 10 atomes de carbone, un résidu alkyle avec 1 à 10 atomes de carbone, un résidu syfamoyle ou un résidu trialkylsilyle.

2. Dérivés de 17α-cyanométhylestra-4,9-diène selon la revendication 1, c'est-à-dire
17α-cyanométhylestra-4,9-dién-3-on-17β-yl-acétate
17α-cyanométhylestra-4,9-dién-3-on-17β-yl-propionate
17α-cyanométhylestra-4,9-dién-3-on-17β-yl-butyrate
17α-cyanométhylestra-4,9-dién-3-on-17β-yl-isobutyrate
17α-cyanométhylestra-4,9-dién-3-on-17β-yl-valérate
17α-cyanométhylestra-4,9-dién-3-on-17β-yl-isovalérate
17α-cyanométhylestra-4,9-dién-3-on-17β-yl-décanoate
17α-cyanométhylestra-4,9-dién-3-on-17β-yl-cyclohexanoate
17α-cyanométhylestra-4,9-dién-3-on-17β-yl-benzoate
17α-cyanométhyl-3-hydroxyiminoestra-4,9-dién-17β-yl-acétate
17α-cyanométhyl-3-hydroxyiminoestra-4,9-dién-17β-yl-propionate
17α-cyanométhyl-3-hydroxyiminoestra-4,9-dién-17β-yl-butyrate
17α-cyanométhyl-3-hydroxyiminoestra-4,9-dién-17β-yl-valérate
17α-cyanométhyl-3-hydroxyiminoestra-4,9-dién-17β-yl-cyclopentanoate
17α-cyanométhyl-3-hydroxyiminoestra-4,9-dién-17β-yl-benzoate
17α-cyanométhyl-3-hydroxyiminoestra-4,9-dién-17β-yl-décanoate
3-acétoxyimino-17α-cyanométhylestra-4,9-dién-17β-yl-acétate
3-acétoxyimino-17α-cyanométhylestra-4,9-dién-17β-yl-valérate
3-acétoxyimino-17α-cyanométhylestra-4,9-dién-17β-yl-benzoate
17α-cyanométhyl-3-propionyloxyimino-estra-4,9-dién-17β-yl-acétate
3-benzoyloxyimino-17α-cyanométhylestra-4,9-dién-17β-yl-acétate
3-benzoyloxyimino-17α-cyanométhylestra-4,9-dién-17β-yl-benzoate
3-tert-butoxyimino-17α-cyanométhyl-estra-4,9-dién-17α-yl-acétate
3-tert-butoxyimino-17α-cyanométhyl-estra-4,9-dién-17β-yl-benzoate
17α-cyanométhyl-3-(N,N-diéthylsulfamoyloxy)-imino-estra-4,9-dién-17β-yl-acétate
17α-cyanométhyl-3-(N,N-diéthylsulfamoyloxy)-imino-estra-4,9-dién-17β-yl-valérianate.

3. Procédé pour la production de dérivés de 17α-cyanométhylestra-4,9-diène selon les revendications 1 et 2,
caractérisé en ce que
l'on estérifie des composés de la formule générale II 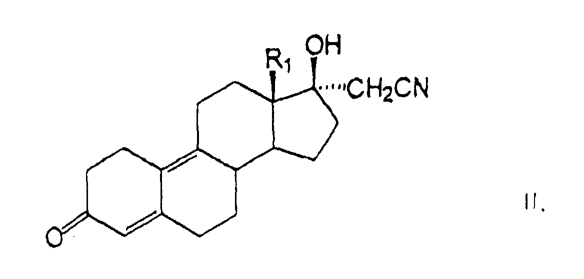 où
R₁ représente un groupe alkyle avec 1 à 2 atomes de carbone,
d'une manière connue avec un anhydride d'acide, un halogénure d'acide en présence d'une amine avec addition d'un catalyseur, en produits de la revendication 1.

4. Procédé pour la production de
dérivés de 17α-cyanométhylestra-4,9-diène selon les revendications 1 et 2,
caractérisé en ce que,
l'on estérifie des composés de la formule générale II
où
R₁ présente les significations indiquées à la revendication 3, d'une manière connue avec un dérivé d'alcénoyl-acyle, en composés de la formule générale III 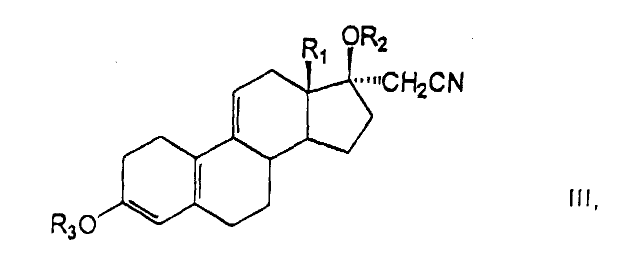 où
R₁ est un groupe alkyle avec 1 à 2 atomes de carbone
R₂, R₃, un résidu acyle avec 1 à 10 atomes de carbone,
et on soumet, dans un solvant organique aqueux, avec addition d'un catalyseur, à une dissociation d'énolester.

5. Procédé pour la production de dérivés de 17α-cyanométhylestra-4,9-diène selon les revendications 1 et 2,
caractérisé en ce que,
l'on transforme des composés de la formule générale IV 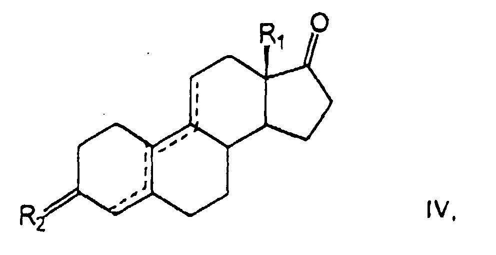 où
R₁ représente un groupe alkyle de 1 à 2 atomes de carbone
R₂, un groupe diméthoxy-, éthylénedioxy-,
2,2-diméthyl-1,3-propylénedioxy,
d'une manière connue, en dérivés 17α-cyanométhyle de la formule générale V,
où
R₁ représente un groupe alkyle avec 1 à 2 atomes de carbone
R₂, un atome d'hydrogène, un atome de lithium
R₃, un groupe diméthoxy-, éthylènedioxy-,
2,2-diméthyl-1,3-propylénedioxy
d'une manière connue, avec un anhydride d'acide ou halogénure d'acide en présence d'une amine avec addition d'un catalyseur,
et on estérifie de façon analogue à la revendication 3
en composés de la formule générale V 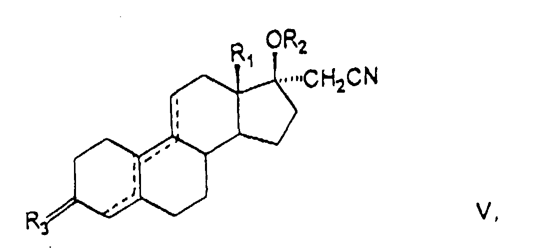 où
R₁ représente un groupe alkyle avec 1 à 2 atomes de carbone
R₂, un résidu acyle avec 1 à 10 atomes de carbone
R₃, un groupe diméthoxy-, éthylénedioxy,
2,2-diméthyl-1,3-propylènedioxy
et on soumet à une dissociation cétal dans un solvant organique aqueux avec addition d'un catalyseur.

6. Procédé pour la production de dérivés de 17α-cyanométhylestra-4,9-diéne
selon les revendications 1 et 2,
caractérisé en ce que
l'on transforme des composés de la formule générale I 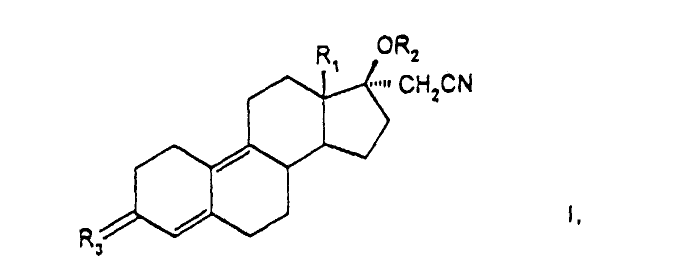 où
R₁ représente un groupe alkyle avec 1 à 2 atomes de carbone
R₂, un résidu acyle avec 1 à 10 atomes de carbone,
R₃, un atome d'oxygène,
d'une manière connue avec du chlorure d'hydoxylammonium dans une amine ou dans un solvant inerte avec addition d'un agent liant l'acide,
et on isole le dérivé d'hydroxyiminostéroïde
où
R₁ et R₂ ont les significations précédemment définies,
R₃ est un groupe hydroxyimino
et/ou on transforme celui-ci de manière connue avec un dérivé d'acide dans une amine ou dans un solvant inerte avec addition d'une amine et on isole le dérivé d'acyloxyimino, le dérivé de sulfamoyloxyimino
où
R₁ et R₂ ont les significations ci-dessus indiquées et
R₃ est un résidu acyloxyimino ou sulfamoyloxyimino
ou bien on réduit l'hydroxyimino produit d'une manière connue avec des alcènes dans des solvants organiques inertes avec addition de catalyseurs
ou bien avec des halogénures de trialkylsilyle dans des solvants organiques inertes avec addition d'une amine, en un dérivé alkyloxyimino ou trialkylsilyloxyimino
où
R₁ et R₂ ont la signification précitée
R₃ est un résidu alcoxyimino ou trialkylsilyloxyimino.

7. Procédé pour la production de dérivés de 17α-cyanométhylestra-4,9-diène
selon les revendications 1 et 2,
caractérisé en ce que
l'on transforme les composés de la formule générale VI 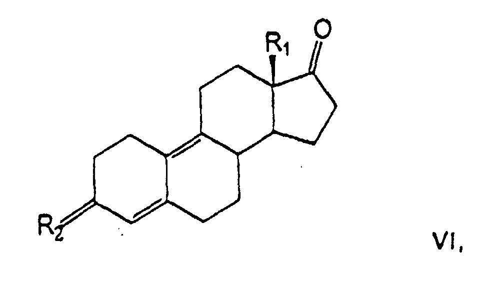 où
R₁ représente un groupe alkyle avec 1 à 2 atomes de carbone,
R₂ est un groupe hydroxyimino,
d'une manière connue avec du lithiumacétonitrile
ou
avec de l'iodure de triméthylsulfonium et ensuite traitement avec un cyanure alcalin, en composés de la formule générale I 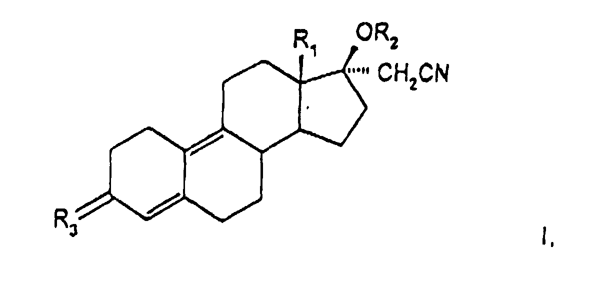 où
R₁ est un groupe alkyle avec 1 à 2 atomes de carbone,
R₂, un atome d'hydrogène et
R₃, un groupe hydroxyimino
et on transforme ceux-ci d'une manière connue avec un dérivé acide dans une amine ou dans un solvant inerte avec addition d'une amine et on isole le dérivé d'acyle
où
R₁ possède la signification précitée et
R₂ est un acyle C₁₋₁₀ et
R₃ est un acyloxyimino C₁₋₁₀

8. Compositions pharmaceutiques contenant au moins un dérivé de 17α-cyanométhylestra-4,9-diène selon la revendication 1 ou 2, le cas échéant, avec des agents auxiliaires et véhicules pharmaceutiquement compatibles.
